# EUROPÄISCHE PATENTSCHRIFT

(11) **EP 0 497 240 B1**
(45) Veröffentlichungstag und Bekanntmachung des Hinweises auf die Patenterteilung: **08.04.1998**
(21) Anmeldenummer: 92101259.7
(22) Anmeldetag: 27.01.1992
(51) Int. Cl.: A61K 9/22

(54) **Zubereitung zur kontrollierten Freigabe von Wirkstoffen für Wiederkäuern**
Preparation for controlled release of drugs for ruminants
Préparation pour la libération contrôlée de principes actifs pour ruminants

(30) Priorität: 28.01.1991 DE 4102395; 23.04.1991 DE 4113146
(43) Veröffentlichungstag der Anmeldung: 05.08.1992
(73) Patentinhaber: HOECHST AKTIENGESELLSCHAFT, 65926 Frankfurt am Main (DE)
(72) Erfinder: Hornykiewytsch, Theophil, Dr., W-6230 Frankfurt am Main 80 (DE); Düwel, Dieter, Dr., W-6238 Hofheim am Taunus (DE)

(56) Entgegenhaltungen:
- EP-A- 0 025 696
- EP-A- 0 025 697
- EP-A- 0 236 002
- EP-A- 0 236 901
- EP-A- 0 284 258
- EP-A- 0 385 106
- EP-A- 0 406 015
- GB-A- 2 077 103
- US-A- 3 535 419
- WORLD PATENTS INDEX Section Ch, Week 8804, Derwent Publications Ltd., London, GB; Class C, AN 88-021787

## Beschreibung

Formling zur kontrollierten Freigabe von Wirkstoffen, die als Therapeutika oder zur Verbesserung des Wachstums und der Futterverwertung bei Wiederkäuern geeignet sind

Die Erfindung betrifft einen, Formling, der mindestens einen Wirkstoff, ein Wachs, ein Beschwerungsmittel und gegebenenfalls einen Zucker, Zuckeralkohol, Celluloseether oder ein Polyethylenglykol enthält, ein Verfahren zu dessen Herstellung und dessen Verwendung in der Tiermedizin und Tierernährung.

Wirkstofffreigabesysteme sind Zubereitungen, die einen oder mehrere (physiologisch) wirksame Bestandteile in einem definierten Freigabeprofil über eine festgesetzte Zeit hinweg kontrolliert freisetzen.

Die wichtigsten Indikationsgebiete zur Anwendung dieser Systeme in der Tiermedizin sind:
1) Therapie im engeren Sinn, d.h. die Kontrolle parasitärer Infektionen und/oder Erkrankungen, sowie im weiteren Sinne auch
2) die Zufuhr von Mangelstoffen (z.B.: Spurenelemente),
3) die Steuerung metabolischer Prozesse (z.B.: Leistung),
4) die Steuerung endokriner Vorgänge (z.B.: Fertilität).

Indikationsgebiete zur Anwendung dieser Systeme in der Tierernährung sind insbesondere die Verbesserung des Wachstums und der Futterverwertung bei Wiederkäuern.

Vorteile der Wirkstoffreigabesysteme im Vergleich zu herkömmlichen Darreichungsformen sind ein optimaler Wirkstoffspiegel während der gesamten Anwendungsdauer, eine niedrigere Wirkstoffdosierung, eine Arbeits- und damit letztendlich Kostenersparnis für den Tierhalter.

Die möglicherweise auftretenden Nachteile, wie Resistenzentwicklung bzw. Gewöhnung, Wirkstoffrückstände und Wartezeit, Gewebeirritationen und Verbleib eines Fremdkörpers nach Anwendungsende können und müssen durch eine optimale galenische Entwicklung ausgeschlossen oder doch zumindest deutlich minimiert werden.

Wirkstoffreigabesysteme zur oralen Applikation bei Wiederkäuern, im folgenden "Bolus" genannt, sind in der Fachliteratur ausführlich beschrieben (z.B. Formulation of Veterinary Dosage Forms, by Jack Blodinger, Marcel Dekker Inc. New York, 1983). Der Verbleib des Bolus im Gastrotrakt des Tieres wird bewirkt durch die geometrische Gestalt oder durch die Dichte des Systems, Im letzteren Fall spricht man von einem "high density device" (HDD). Aus der Literatur ist bekannt, daß HDD eine Dichte > 2 g/cm³ haben sollten (J. Riner et al., Am. J. Vet. Res. 43 (1982), S. 2028-2030), damit der Bolus trotz der natürlichen Regurgitation des Wiederkäuers nicht wieder hervorgewürgt werden kann.

Die bisher beschriebenen "high density devices" sind daher mit einem Gewichtselement versehen, um die benötigte Gesamt-Dichte zu erreichen. Dieses Gewichtselement wird bevorzugt als massiver Metallblock z.B. EP-A-0 333 311, EP-A-0 149 510, EP-A-0 062 391 oder als Zylinder z.B. EP-A-0 164 241 und Vet. Parasitology 12 (1983) 223-232 ausgeführt. Diese in der Literatur beschriebenen Metallelemente haben den Nachteil, daß sie nicht bioabbaubar sind und über die gewünschte Applikationsdauer hinaus als Fremdkörper im Magen des Tieres verbleiben. Nachteilige Folgen davon sind u.a. Interaktionen mit anderen Bolus-Systemen bei mehrfacher Applikation, Irritationen der Schleimhäute und Probleme beim Schlachten und Zerlegen der Tiere.

Einen gewissen Fortschritt zur Überwindung dieser Nachteile bietet die Verwendung abbaubarer Gewichtselemente, wie in EP-A-0 332 094 beschrieben. Allerdings sind auch die dort beschriebenen Systeme noch nicht optimal geeignet. Sie sind aus mehreren ganz unterschiedlichen Komponenten zusammengesetzt. Diese Komponenten sind u.a. a) eine Mischung (Matrix) aus einem abbaubaren Polymer und Wirkstoff, b) eine Mischung (Matrix) aus einem abbaubaren Polymer und Teilchen hoher Dichte, c) ein abbaubares Polymer und d) Teilchen hoher Dichte.

Nachteilig ist der aufwendige Aufbau der Systeme, der im Regelfall eine getrennte Herstellung mit späterem Zusammenfügen der Einzelteile erforderlich macht. Die maximal zu applizierende Wirkstoffmenge wird durch das begrenzte Volumen des abbaubaren Polymers stark eingeschränkt. Ferner ist zu beachten, daß durch Abbau der verschiedenen Polymere oder durch mechanische Einflüsse im Gastrotrakt leicht eine Separierung der einzelnen Teile des Systems erfolgen kann. Die beschriebenen Bolus-Systeme entsprechen daher nur mit Einschränkungen den Erfordernissen der Praxis in Herstellung und therapeutischer Sicherheit.

In der EP-A-0 243 111 werden schließlich u.a. Bolus-Systeme beschrieben, die aufgebaut sind aus Formkörpern, die neben dem Wirkstoff ein Eisen-Granulat und Graphit enthalten sowie eine Hülle aus Magnesium-Legierung und eine Schutzhülle aus nicht abbaubarem Kunststoff. Magnesium, Eisen und Graphit bilden im Magensaft ein galvanisches Element, das die Korrosion der Magnesium-Hülle bewirkt. Auf diese Weise wird die Wirkstoff-Freisetzung gesteuert.

Nachteilig an diesem System ist die gleichzeitige Freisetzung von Magnesium-Ionen. Magnesium ist eine physiologisch wirksame Substanz und wird in der Veterinärmedizin als Arzneistoff, z.B. gegen Gras-Tetanie bei Kälbern, eingesetzt.

Die Fertigung der obengenannten Bolus-Systeme ist vergleichsweise aufwendig und kostenintensiv. Das Verpressen des Eisen-Granulates zu den Formlingen bewirkt eine erheblicher Abrieb der verwendeten Preßwerkzeuge. Die daraus resultierenden kurzen Standzeiten führen zu einer Erhöhung der Fertigungskosten. Die Verwendung eines nicht abbaubaren Kunststoffes ist nicht unproblematisch, da der Kunststoff nach Ende der Wirkdauer des Bolus entweder als Fremdkörper im Tier verbleibt oder aber ausgeschieden wird und die Umwelt belastet.

Ein praxisgerechtes Bolus-System sollte folgende Forderungen erfüllen:
1) einfache, sichere Applizierbarkeit,
2) vollständige Retention am Wirkort während der gesamten Dauer der Wirkstofffreisetzung,
3) kontrollierte Wirkstoff-Freisetzung mit klarem Endpunkt,
4) keine unerwünschten Nebenwirkungen und keine Gewebeschäden,
5) kein verbleibender Fremdkörper nach Abschluß der Behandlung.

Diese Forderungen werden überraschenderweise von einem Bolus-System erfüllt, bei welchem die Bolus-Zubereitung aus dem Wirkstoff, einem nicht wasserlöslichen Paraffin oder Wachs, einem Stoff der die mechanischen Eigenschaften der Zubereitung, insbesondere Abrieb und/oder Zerfall beeinflußt, ggf. einem oberflächenaktiven Stoff und einem pulverförmigen Stoff hoher Dichte (> 3 g/cm³) besteht.

Die vorliegende Erfindung betrifft daher einen Formling zur oralen Applikation bei Wiederkäuern, enthaltend 0,001 bis 75 Gew.-% mindestens eines Wirkstoffs, 3 bis 75 Gew.-% Wachs, 25 bis 90 Gew.-% pulverförmiges Beschwerungsmittel und 0 bis 30 Gew.-% mindestens eines physiologisch verträglichen Zuckers, Zuckeralkohols, wasserlöslichen Celluloseethers oder Polyethylenglykols herstellbar, indem die Bestandteile des Formlings in einem Mischer derart gemischt werden, daß die Friktionswärme zur Ausbildung eines Schmelzgranulats führt, das nach dem Abkühlen ohne weiteres Schmelzen zu einem Formling verpreßt wird.

Vorzugsweise enthält der Formling bis zu 50 Gew.-%, insbesondere bis zu 30 Gew.-% mindestens eines Wirkstoffs, vorzugsweise 5 bis 60 Gew.-%, insbesondere 5 bis 50 Gew.-% Wachs, vorzugsweise 30 bis 85 Gew.-%, insbesondere 40 bis 80 Gew.-% pulverförmiges Beschwerungsmittel und vorzugsweise 0 - 14 Gew.-%, insbesondere 0 - 10 Gew.-% mindestens eines physiologisch verträglichen Zuckers, Zuckeralkohols, wasserlöslichen Celluloseethers oder Polyethylenglykols, oder deren Gemisch.

Falls ein Wirkstoff nicht oder nur in geringem Maße wasserlöslich ist (wie z.B. Fenbendazol), ist es vorteilhaft, den Gehalt an Zucker, Zuckeralkohol, wasserlöslichem Celluloseether oder Polyethylenglykol nicht unter 1 Gew.-% zu senken. Bevorzugt ist dann ein Gehalt von 1,5 bis 27 Gew.-%, insbesondere 2 bis 25 Gew.-%.

Der Formling kann außerdem noch bis zu 8 Gew.-%, vorzugsweise bis zu 6 Gew.-% eines oberflächenaktiven Stoffes, bis zu 10 Gew.-%, vorzugsweise bis zu 6 Gew.-% Formentrennmittel, ein Schmiermittel und/oder Stoffe, welche die Eigenschaften der Zubereitung beeinflussen, enthalten.

Unter Wiederkäuern werden insbesondere Rinder, Schafe und Ziegen verstanden.

Bei den therapeutischen Wirkstoffen handelt es sich insbesondere um Mittel zur Kontrolle parasitärer Infektionen und/oder Erkrankungen, zur Zufuhr von Mangelstoffen, zur Steuerung metabolischer Prozesse oder zur Steuerung endokriner Vorgänge.

Erfindungsgemäß können beispielsweise die nachfolgend genannten, therapeutisch wirksamen Stoffgruppen und Verbindungen in dem Formling eingesetzt werden.
- Glucocorticoide zur Geburtsinduktion, z.B. Dexamethason, Betamethason, Flumethason, deren Ester und Derivate,
- Gestagene zur Brunstsynchronisation, Brunst- und Läufigkeitsunterdrückung,
- β₂-Adrenergika zur Therapie und Prophylaxe von Respirationserkrankungen, zur Verhinderung von Abort und Geburt, zur Wachstumsförderung und Stoffwechselbeeinflussung, wie z.B. Clenbuterol, 4-(2-tert.-Butylamino-1-hydroxyethyl)-2-cyano-6-fluor-phenylcarbaminsäure-ethylester-hydrochlorid, α[[[3-(1-Benzimidazolyl)-1,1-dimethylpropyl]-amino]-methyl]-2-fluor-4-hydroxybenzyl alkohol-methansulfonat monohydrat (Cimaterol), 1-(4-Amino-3-cyanophenyl)-2-iso-propylaminoethanol,
- β-Blocker zur Reduzierung von Transport-Stress, α₂-Adrenergika gegen enteritische Erkrankungen und zur Behandlung hypoglykämischer Zustände, sowie zur Sedation (z.B. Clonidin), 2-[2-Brom-6-fluorphenylimino]-imidazolidin,
- Benzodiazepine und Derivate, wie z.B. Brotizolam zur Sedation,
- Antiphlogistika zur antiinflammatorischen Therapie, z.B. Meloxicam
- Endorphine zur Anregung der Pansenmotorik,
- Steroidhormone (natürliche und synthetische) zur Wachstumsförderung, z.B. Östradiol, Progesteron und deren Ester und synthetische Derivate wie z.B. Trenbolon,
- Antiparasitika zur Bekämpfung von Endo- und Ektoparasiten, wie z.B. Levamisol, Avermectin, Benzimidazole, Pyrantel, Morantel, Febantel,
- herz- und kreislaufaktive Substanzen, z.B. Etilefrin oder Pimobendan.

Bevorzugt sind Wirkstoffe aus der Gruppe der Benzimidazol-, Benzthiazol-Derivate oder der Pro-Benzimidazole, insbesondere Verbindungen der Formeln I, II, oder III 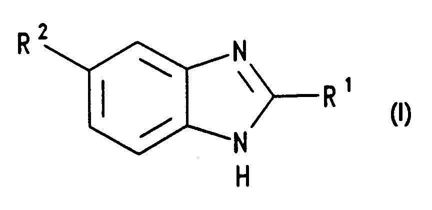
Ia), worin
   - R¹: Methoxycarbonylamino bedeutet und
   - R²: n-Propylmercapto (Albendazol), Phenylmercapto (Fenbendazol), Phenylsulfinyl (Oxfendazol), Benzoyl (Mebendazol), p-Fluorbenzoyl (Flubendazol), p-Fluorphenylsulfonyloxy (Luxabendazol), Cyclopropylcarbonyl (Cyclobendazol), n-Butyl (Parbendazol), n-Propoxy (Oxibendazol) oder H (Carbendazim) bedeutet oder
Ib), worin
   - R¹: 4-Thiazolyl bedeutet und
   - R²: H (Thiabendazol) oder Isopropoxycarbonylamino (Cambendazol).

### II.

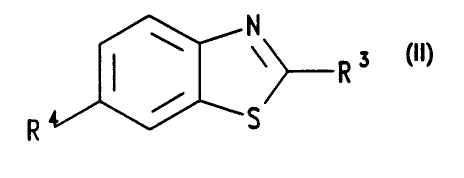

worin
- R³: Methoxycarbonylamino bedeutet und
- R⁴: n-Propoxy bedeutet, (Tioxidazol).

### III.

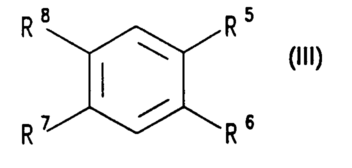

worin
- R⁵: -N=C(NHCOOCH₃)₂,
- R⁶: -NHCOCH₂OCH₃,
- R⁷: Phenylmercapto und
- R⁸: H bedeuten (Febantel); oder
- R⁵: 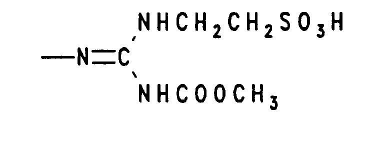
- R⁶: NO₂, R⁷ H und R⁸ n-Propylmercapto bedeuten (Netobimin); oder
- R⁵ und R⁶: jeweils -NHCSNH-COOC₂H₅, R⁷ und R⁸ jeweils H bedeuten (Thiophanat).

Besonders bevorzugt ist Fenbendazol.

Zur Verbesserung des Wachstums und der Futterverwertung bei Wiederkäuern können beispielsweise die nachfolgend genannten Stoffgruppen und Verbindungen in dem erfindungsgemäßen Formling eingesetzt werden.

Salinomycin, Flavophospholipol, Monensin, Dehydromethylmonensin, Narsin, Desoxynarsin, Lasalocid A, Alborixin, Lysocellin, Nigericin, Dehydroxymethylnigericin, Dianemycin, Ionomycin, Norbitomycin und andere Polyetherantibiotika, Avoparcin, Spiramycin, Tylosin, Virginiamycin, Bacitracin A, Carbadox, Nitrovin, Olaquindox, Amprolium, Arprinacid, Dinitolmid, Halofuginon, Metichlorpindol, Nicarbazin, Decoquinate, Triazinderivate und die Salze der genannten Verbindungen wie Salinomycin-Na, Monensin-Na, Tylosin-Phosphat, Zn-Bacitracin A, Lasalocid A-Na.

Besonders bevorzugt sind Phospho-Glykolipide, insbesondere Flavophospholinol, Salinomycin sowie dessen Salz, vorzugsweise Na-Salz.

Unter einem Wachs im Sinne der vorliegenden Erfindung versteht man einen physiologisch unbedenklichen natürlich oder künstlich gewonnenen Stoff, der folgende Eigenschaften aufweist: Bei Raumtemperatur fest bis brüchig hart, grob- bis feinkristallin, durchscheinend bis opak, jedoch nicht glasartig; über 40°C ohne Zersetzung schmelzend, schon wenig oberhalb des Schmelzpunktes verhältnismäßig niedrigviskos und nicht fadenziehend, stark temperaturabhängige Konsistenz und Löslichkeit.

Von ähnlichen synthetischen oder natürlichen Produkten (z.B. Harzen, plastischen Massen) unterscheiden sie sich hauptsächlich darin, daß sie in der Regel etwa zwischen 50 und 90°C schmelzen. Geeignet sind z.B. rezente Wachse, wie Candelilla-, Carnauba-Wachs, und fossile Wachse wie Montanwachs usw., Mineralwachse wie Ceresin, Ozokerit (Erdwachs),

Petrolatum, Paraffin- und Mikro-Wachse; chemisch veränderte Wachse sind z.B. die durch Oxidation von Rohmontanwachs hergestellten Hoechst- und BASF-Wachse und hydriertes Jojoba-Öl oder Rizinus-Öl (z.B. Cutina HR) sowie synthetische Wachse (Kunstwachse).

Das pulverförmige Beschwerungsmittel ist bezüglich seiner spezifischen Dichte, seiner Zusammensetzung und seiner Menge so auszuwählen, daß das Bolus-System trotz der natürlichen Regurgitation im Magen verbleibt. In Abhängigkeit von der Größe des Bolus-Systems und seiner Oberfläche ist die spezifische Dichte je nach Zusammensetzung des dichtegebenden Teiles im allgemeinen größer als 3,0 g/cm³, vorzugsweise größer als 4,0 g/cm³.

Nach oben stellt die Dichte kein einschränkendes Kriterium dar.

Beispielhaft werden Beschwerungsmittel aufgeführt, welche eine ausreichend hohe Dichte aufweisen:

| Beschwerungsmittel | Dichte (g/cm³ |
|---|---|
| Silber | 10,5 |
| Kupfer | 8,9 |
| Eisen | 7,8 |
| Nickel | 8,9 |
| Blei | 11,3 |
| Antimon | 6,7 |
| Zinn | 7,3 |
| Zink | 7,1 |
| Hydroxylapatit | 3,1-3,3 |
| Bariumsulfat | 4,5 |
| Eisenoxide | 5,2-5,7 |
| Bariumtitanat | 6,1 |
| Aluminiumoxid | 4,0 |
| Zinnoxid | 7,0 |
| Titandioxid (Rutil) | 4,2 |
| Scheelit (Calciumwolframat | 6,1 |
| Ferberit (Eisenwolframat) | 7,2 |
| Fayalit (Eisensilicat) | 4,4 |
| Hercynit (Eisenaluminiumoxid) | 4,3 |
| Powellit (Calciummolybdat) | 4,3 |
| Calciumphosphate | 4,3 |

Bevorzugt eingesetzt werden nicht toxische Stoffe, wie Bariumsulfat oder Eisenpulver, insbesondere Eisenpulver.

Bei Stoffen hoher spezifischer Dichte werden Teilchengrößen kleiner als 1 mm, eventuell auch kleiner als 0,1 mm bevorzugt. So ist bei dem erfindungsgemäß verwendeten Eisenpulver 97 % der Teilchen ≤ 0,15 mm, 80 % ≤ 0,1 mm.

Bei wasserlöslichen Wirkstoffen kann der Anteil an Zucker, Zuckeralkohol, wasserlöslichem Celluloseether oder Polyethylenglykol reduziert werden, oder diese Hilfsstoffe können ganz entfallen.

Erfindungsgemäß geeignete Zucker sind beispielsweise wasserlösliche pharmazeutisch akzeptable Monosaccharide und Disaccharide. Bevorzugt wird Lactose, geeignet sind jedoch auch Glucose, Fructose, Xylose, Galactose, Sucrose, Maltose und verwandte Verbindungen wie Mannit, Xylit, Sorbit.

Geeignete wasserlösliche Celluloseether sind die ®Tylose-Marken ®Tylose MB (Methylcelluose) und ®Tylose H (Hydroxyethylcellulose), bevorzugt ist Methylcellulose.

Polyethylenglykole (PEG) im Sinne der vorliegenden Erfindung sind z.B. PEG 400, 600, 1500, 2000, 3000, 4000, 6000 und 10000. Bevorzugt sind die festen Typen.

Als oberflächenaktive Stoffe kommen bevorzugt physiologisch verträgliche nichtionische Tenside mit einem HLB-Wert zwischen 10 und 20, insbesondere die Polyoxyethylen-sorbitan-Ester, wie das Monolaurat (®Tween 20), das Monopalmitat (®Tween 40) und das Monostearat (®Tween 60) in Frage. Bevorzugt ist ®Tween 20.

Als Schmiermittel und als Formentrennmittel werden üblicherweise Talk, Stearate oder andere Metallseifen, wie Fumarate oder Palmitate, eingesetzt, bevorzugt Magnesiumstearat.

Als Hilfsstoff, der die mechanischen Eigenschaften, insbesondere Abrieb und Zerfall, beeinflussen kann, wird üblicherweise hochdisperses Siliciumdioxid (®Aerosil) eingesetzt.

Die Erfindung betrifft weiterhin Formlinge zur oralen Applikation bei Wiederkäuern bestehend aus einem oben definierten Mittel, welches eine für eine definierte Freigabe während einer festgesetzten Zeit ausreichende Menge des Wirkstoffs enthält, und einer im Verdauungstrakt löslichen oder abbaubaren Hülle.

Weiterhin betrifft die vorliegende Erfindung ein Verfahren zur Herstellung eines wie zuvor beschriebenen Formlings, dadurch gekennzeichnet, daß man aus den Bestandteilen des Formlings in einem geeigneten Mischer unter Ausnutzung der Friktionswärme ein Schmelzgranulat herstellt, das nach dem Abkühlen ohne weiteres Schmelzen zu einem Formling verpreßt wird.

Der Formling bzw. die einzelnen Formlinge haben je nach Tierart einen Durchmesser, der zwischen 1 cm und 4 cm liegt. Die Gesamtlange des Bolus sollte 12 cm nicht überschreiten, die untere Grenze ist eine einzelne Tablette. Der Formling kann aus einem Stück (Fig. 1) oder einer beliebigen Anzahl von Einzelstücken (Fig. 2 und 3) bestehen.

Das Gesamtvolumen kann damit zwischen etwa 0,5 und 200 cm³ liegen.

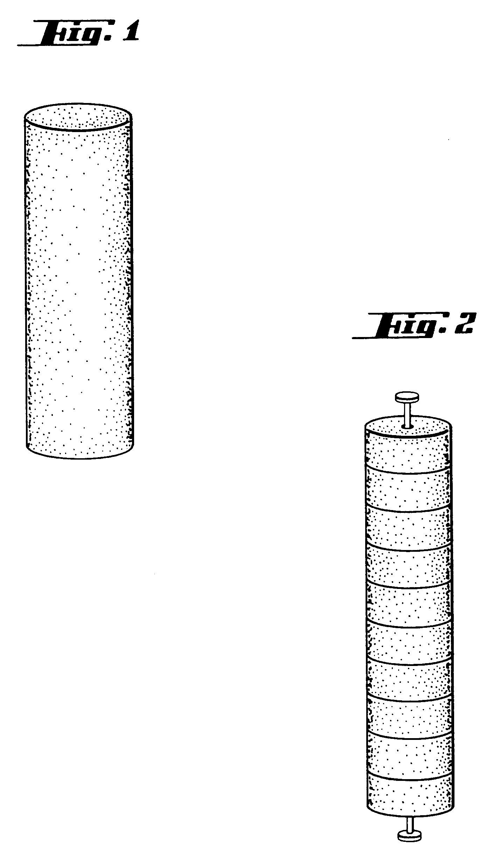

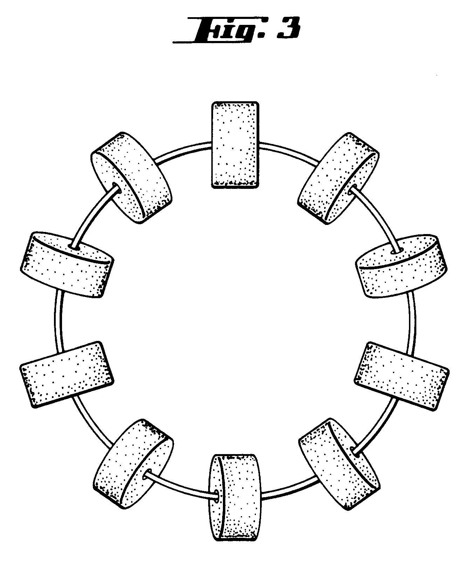

Durch Vergrößerung der Oberfläche, d.h. durch Übergang von einem Stück (Fig. 1) zu der Tablettenform (Fig. 2, 3) kann die Freisetzungsrate bei gleicher Zusammensetzung der Matrix gesteigert werden und je nach Anzahl, Größe und Form der Formlinge den Erfordernissen angepaßt werden. Der Verlust einzelner Formlinge während der Behandlungszeit, z.B. durch Regurgitation, wird verhindert, indem die Tabletten miteinander verbunden werden. Diese Verbindung kann flexibel, z.B. durch ein Band, einen Faden oder eine Kette, elastisch oder starr, z.B. durch einen Stab, erfolgen (Fig. 2, 3). Das Verbindungsmaterial kann beispielsweise aus physiologisch unbedenklichem Kunststoff (z.B. Silicon) oder aus einem physiologisch unbedenklichen Material sein, das im Darm abgebaut wird.

Ein Tabletten-Formling (Fig. 2, 3) vermeidet außerdem die Gefahr, daß die Matrix durch äußere Einwirkung zerbrechen bzw. beschädigt werden kann. So wird eine gleichmäßigere Freisetzung während der gesamten Applikationsdauer gewährleistet.

Die Höhe der Formlinge kann abhängig vom gewünschten Freisetzungsprofil des Wirkstoffes variieren. Ebenfalls sind verschiedene Tablettenformen, z.B. biplan, bikonvex, möglich. Es können in einem Bolus auch Formlinge unterschiedlicher Hohe und Form miteinander kombiniert werden. Ebenfalls ist es möglich, Formlinge die mit einem Wirkstoff in unterschiedlicher Konzentration oder mit verschiedenen Wirkstoffen beladen sind, miteinander zu kombinieren. Zur Erleichterung der Applikation können die miteinander verbundenen Formlinge mit Hilfe eines wasserlöslichen Klebstoffes zusammen geklebt oder mit einer im Verdauungstrakt löslichen oder abbaubaren Hülle umgeben werden. Das Material des Klebestoffes bzw. der Hülle kann z.B. aus Cellulose und Cellulose-Derivaten, Gelatine und anderen geeigneten Polymeren und Copolymeren bestehen. Die Formlinge können auch von einem Netz umgeben sein.

Die in der obengenannten Weise hergestellten Erzeugnisse können aus bis zu 30, vorzugsweise bis zu 20, insbesondere bis zu 10 Formlingen bestehen. Die Matrix wird während der Behandlungszeit vollständig abgebaut; ein Rückstand bleibt nach Abschluß der Behandlung nicht zurück.

Das beschriebene Wirkstofffreigabesystem ist grundsätzlich geeignet für die Applikation aller Wirkstoffe, die in den Bereichen Veterinärmedizin und Tierernährung über einen längeren Zeitraum verabreicht werden. Unterschiedliche physikalische Eigenschaften der Wirkstoffe, z.B. Löslichkeitsunterschieden, können durch geeignete Zusatzstoffe, die die Wirkstofffreisetzung steuern, beeinflußt werden. Geeignete Zusatzstoffe zur Steuerung des Freisetzungs-Profils entsprechend den therapeutischen Anforderungen sind dem Fachmann bekannt.

Die Erfindung betrifft daher auch die Verwendung der obengenannten Formlinge und Erzeugnisse bei Wiederkäuern zur Prophylaxe und/oder Behandlung von Krankheiten sowie zur Beeinflussung des Wachstums, des Stoffwechsels, des Körpergewichts, der Gewebezusammensetzung und/oder der Futterverwertung.

Die nachfolgenden Beispiele dienen zur Erläuterung der Erfindung, ohne daß diese darauf beschränkt wären.

### B. Allgemeines Herstellungsbeispiel für ein Schmelzgranulat

Die Bestandteile der Formulierung werden in einen geeigneten Mischer (z.B. Fluidmischer Fa. Henschel) eingewogen. Die durch die Friktion beim Mischen entstehende Wärme führt zu Ausbildung eines Schmelzgranulates. Nach Abkühlen des Granulates auf Raumtemperatur werden Agglomerate durch Passieren über ein Sieb (z.B. Frewitt Granulator, Maschenweite: 1 mm) zerkleinert.

Das so standardisierte Granulat wird mit einer Presse zu den gewünschten Formlingen verpreßt. Bei Bedarf kann ein Schmiermittel bzw. Formentrennmittel zugegeben werden.

Die in den folgenden Beispielen nach A. oder B. hergestellten Zubereitungen haben vorzugsweise folgende Zusammensetzung (%-Angaben sind Gew.-%):
Wirkstoff: bis zu 30 %
Wachs: 5 - 50 %
Eisen: 40 - 80 %
Lactose, Methylcellulose oder Polyethylenglykol: 0 - 25 %
hochdisperses Siliziumdioxid: bis zu 2 %
Polyoxyethylen-Sorbitanmonolaurat: bis zu 6 %
Mg-Stearat: bis zu 6 %.

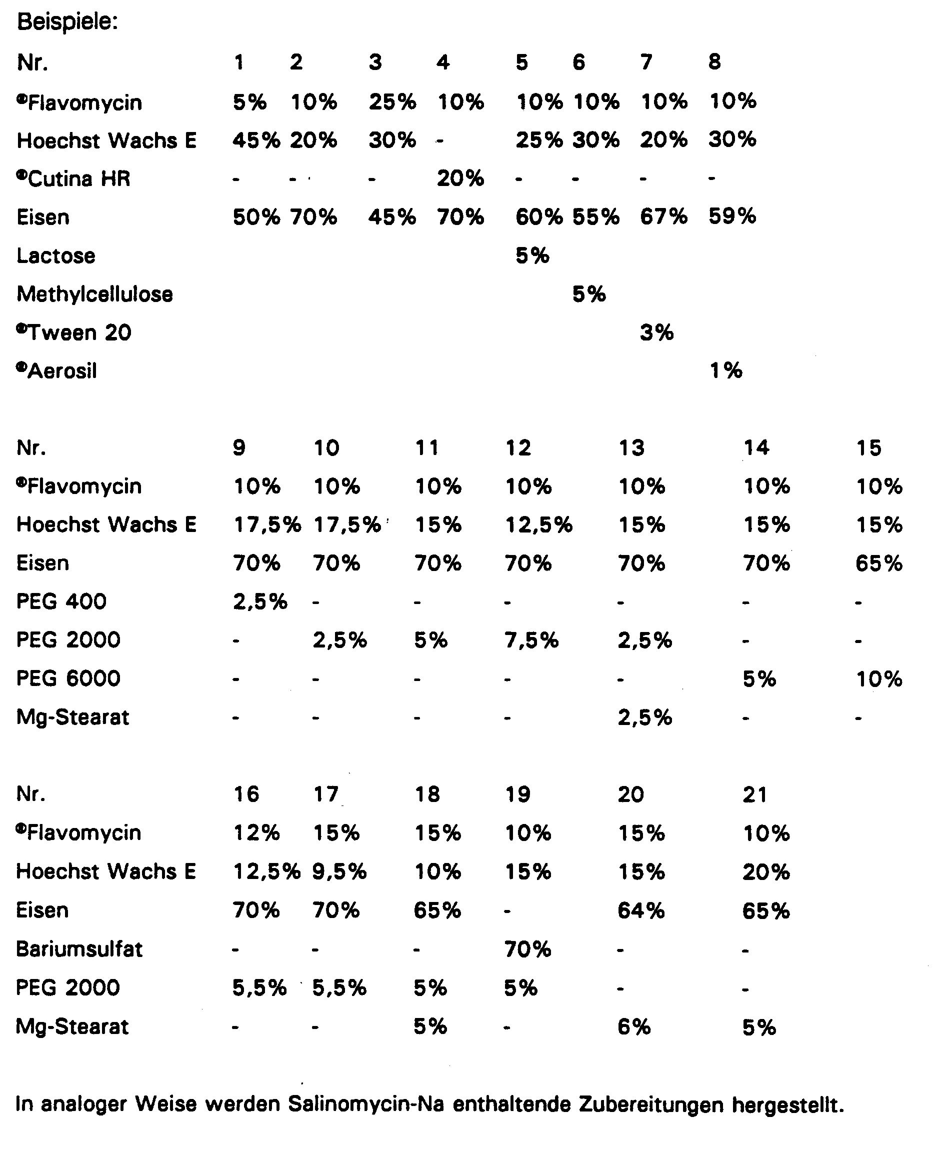

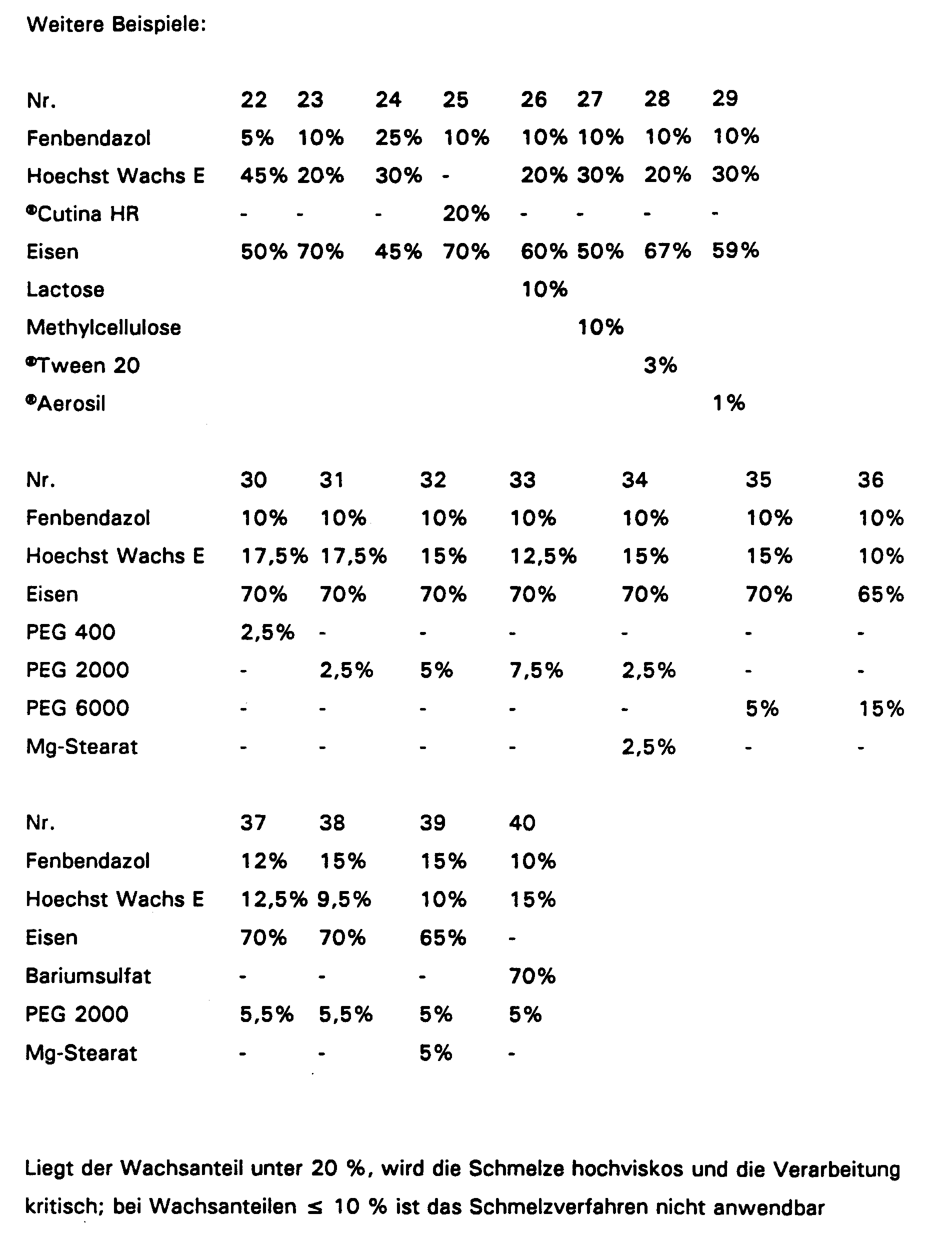

## Patentansprüche (Patentansprüche für folgende(n) Vertragsstaat(en): AT, BE, CH, DE, DK, FR, GB, IT, LI, LU, NL, PT, SE)

1. Formling zur oralen Applikation bei Wiederkäuern, enthaltend 0,001 bis 75 Gew.-% mindestens eines Wirkstoffs, 3 bis 75 Gew.-% Wachs, 25 bis 90 Gew.-% pulverförmiges Beschwerungsmittel und 0 bis 30 Gew.- % mindestens eines physiologisch verträglichen Zuckers, Zuckeralkohols, wasserlöslichen Celluloseethers oder Polyethylenglykols herstellbar, indem die Bestandteile des Formlings in einem Mischer derart gemischt werden, daß die Friktionswärme zur Ausbildung eines Schmelzgranulats führt, das nach dem Abkühlen ohne weiteres Schmelzen zu einem Formling verpreßt wird.

2. Formling gemäß Anspruch 1, dadurch gekennzeichnet, daß er mindestens einen oberflächenaktiven Stoff, ein Schmiermittel, ein Formentrennmittel und/oder einen Stoff, welcher die mechanischen Eigenschaften der Zubereitung beeinflußt, enthält.

3. Formling gemäß Anspruch 1 oder 2, dadurch gekennzeichnet, daß das Beschwerungsmittel Eisenpulver ist.

4. Formling gemäß einem der Ansprüche 1 bis 3, dadurch gekennzeichnet, daß der Wirkstoff ein Mittel zur Kontrolle parasitärer Infektionen und/oder Erkrankungen, zur Zufuhr von Mangelstoffen, zur Steuerung metabolischer Prozesse oder zur Steuerung endokriner Vorgänge ist.

5. Formling gemäß einem der Ansprüche 1 bis 3, enthaltend mindestens einen Wirkstoff zur Verbesserung des Wachstums und/oder der Futterverwertung.

6. Formling gemäß einem der Ansprüche 1 bis 4, dadurch gekennzeichnet daß der Wirkstoff ein Anthelminthikum vorzugsweise aus Wirkstoffgruppe der Benzimidazol-, Benzthiazol-Derivate oder der Pro-Benzimidazole ist.

7. Formling gemäß Anspruch 6, enthaltend den Wirkstoff Fenbendazol.

8. Formling gemäß einem der Ansprüche 1 bis 3 und 5, dadurch gekennzeichnet, daß der Wirkstoff ein Polyetherantibiotikum ist.

9. Formling gemäß einem der Ansprüche 1 bis 3 und 5, dadurch gekennzeichnet, daß der Wirkstoff ein Phospho-Glykolipid ist.

10. Formling gemäß einem der Ansprüche 1 bis 3 und 5, enthaltend den Wirkstoff Salinomycin oder dessen Salz.

11. Formling gemäß Anspruch 9, dadurch gekennzeichnet, daß der Wirkstoff Flavophospholipol ist.

12. Formling zur oralen Applikation bei Wiederkäuern, gemäß einem der Ansprüche 1 bis 11, welcher eine für eine definierte Freigabe während einer festgesetzten Zeit ausreichende Menge des Wirkstoffs enthält und gegebenenfalls einer im Verdauungstrakt löslichen oder abbaubaren Hülle.

13. Erzeugnis enthaltend mehrere Formlinge gemäß Anspruch 12.

14. Erzeugnis gemäß Anspruch 13, dadurch gekennzeichnet, daß die Formlinge mittels eines Bandes, eines Fadens, einer Kette, eines Stabes, durch Verklebung oder Einschluß in ein Netz miteinander verbunden sind.

15. Erzeugnis gemäß Anspruch 13 oder 14, dadurch gekennzeichnet, daß es aus Formlingen unterschiedlicher Abmessung, Form, eines unterschiedlichen Gehalts an Wirkstoff und/oder unterschiedlicher Art des Wirkstoffs bestehen.

16. Erzeugnis gemäß einem der Ansprüche 13 bis 15, dessen Gesamtvolumen zwischen 0,5 und 200 cm³ beträgt.

17. Verfahren zur Herstellung eines Formlings gemäß einem der Ansprüche 1 bis 11, dadurch gekennzeichnet, daß man
aus den Bestandteilen des Formlings in einem geeigneten Mischer unter Ausnutzung der Friktionswärme ein Schmelzgranulat herstellt, das nach dem Abkühlen ohne weiteres Schmelzen zu einem Formling verpreßt wird.

18. Verfahren zur Herstellung von Formlingen gemäß Anspruch 12, dadurch gekennzeichnet, daß man
ein gemäß Anspruch 17 hergestelltes Schmelzgranulat zerkleinert und mit einer Presse zu den gewünschten Formlingen verpreßt
und die so erhaltenen Formlinge mit einer im Verdauungstrakt löslichen oder abbaubaren Hülle vorsieht.

19. Formling gemäß Anspruch 12 oder eines Erzeugnis gemäß einem der Ansprüche 13 bis 16 zur Prophylaxe und/oder zur Behandlung von Krankheiten bei Wiederkäuern.

## Patentansprüche (Patentansprüche für folgende(n) Vertragsstaat(en): ES, GR)

1. Verfahren zur Herstellung eines Formlings zur oralen Applikation bei Wiederkäuern, enthaltend 0,001 bis 75 Gew.-% mindestens eines Wirkstoffs, 3 bis 75 Gew.-% Wachs, 25 bis 90 Gew.-% pulverförmiges Beschwerungsmittel und 0 bis 30 Gew.-% mindestens eines physiologisch verträglichen Zuckers, Zuckeralkohols, wasserlöslichen Celluloseethers oder Polyethylenglykols, dadurch gekennzeichnet, daß man aus den Bestandteilen des Formlings in einem geeigneten Mischer unter Ausnutzung der Friktionswärme ein Schmelzgranulat herstellt, das nach dem Abkühlen ohne weiteres Schmelzen zu einem Formling verpreßt wird.

2. Verfahren gemäß Anspruch 1, dadurch gekennzeichnet, daß ein Formling hergestellt wird, welcher mindestens einen oberflächenaktiven Stoff, ein Schmiermittel, ein Formentrennmittel und/oder einen Stoff, welcher die mechanischen Eigenschaften der Zubereitung beeinflußt, enthält.

3. Verfahren gemäß Anspruch 1 oder 2, dadurch gekennzeichnet, daß man als Beschwerungsmittel Eisenpulver einsetzt.

4. Verfahren gemäß einem der Ansprüche 1 bis 3, dadurch gekennzeichnet, daß man als Wirkstoff ein Mittel zur Kontrolle parasitärer Infektionen und/oder Erkrankungen, zur Zufuhr von Mangelstoffen, zur Steuerung metabolischer Prozesse oder zur Steuerung endokriner Vorgänge einsetzt.

5. Verfahren gemäß einem der Ansprüche 1 bis 3, bei welchem man als Wirkstoff mindestens einen Wirkstoff zur Verbesserung des Wachstums und/der der Futterverwertung einsetzt.

6. Verfahren gemäß einem der Ansprüche 1 bis 4, dadurch gekennzeichnet, daß man als Wirkstoff ein Anthelminthikum vorzugsweise aus Wirkstoffgruppe der Benzimidazol-, Benzthiazol-Derivate oder der Pro-Benzimidazole einsetzt.

7. Verfahren gemäß Anspruch 6, bei welchem der Wirkstoff Fenbendazol ist.

8. Verfahren gemäß einem der Ansprüche 1 bis 3 und 5, dadurch gekennzeichnet, daß der Wirkstoff ein Polyetherantibiotikum ist.

9. Verfahren gemäß einem der Ansprüche 1 bis 3 und 5, dadurch gekennzeichnet, daß der Wirkstoff ein Phospho-Glykolipid ist.

10. Verfahren gemäß einem der Ansprüche 1 bis 3 und 5, dadurch gekennzeichnet, daß der Wirkstoff Salinomycin oder dessen Salz ist.

11. Verfahren gemäß Anspruch 9, dadurch gekennzeichnet, daß der Wirkstoff Flavophospholipol ist.

12. Verfahren zur Herstellung eines Formlings zur oralen Applikation bei Wiederkäuern, gemäß einem der Ansprüche 1 bis 11, welcher eine für eine definierte Freigabe während einer festgesetzten Zeit ausreichende Menge des Wirkstoffs enthält dadurch gekennzeichnet, daß man den Formling mit einer im Verdauungstrakt löslichen oder abbaubaren Hülle versieht.

13. Verfahren gemäß Anspruch 12, bei welchem man mehrere der so hergestellten Formlinge zu einem Erzeugnis verbindet.

14. Verfahren gemäß Anspruch 13, dadurch gekennzeichnet, daß die Formlinge mittels eines Bandes, eines Fadens, einer Kette, eines Stabes, durch Verklebung oder Einschluß in ein Netz miteinander verbunden werden.

15. Verfahren gemäß Anspruch 13 oder 14, dadurch gekennzeichnet, daß Formlingen unterschiedlicher Abmessung, Form, eines unterschiedlichen Gehalts an Wirkstoff und/oder unterschiedlicher Art des Wirkstoffs eingesetzt werden.

16. Verfahren gemäß einem der Ansprüche 13 bis 15, dadurch gekennzeichnet, daß das Gesamtvolumen des so hergestellten Erzeugnisses zwischen 0,5 und 200 cm³ beträgt.

## Claims (Claims for the following Contracting State(s): AT, BE, CH, DE, DK, FR, GB, IT, LI, LU, NL, PT, SE)

1. A molded article for oral administration in ruminants, containing 0.001 to 75% by weight of at least one active substance, 3 to 75% by weight of wax, 25 to 90% by weight of powdered weighting agent and 0 to 30% by weight of at least one physiologically tolerable sugar, sugar alcohol, water-soluble cellulose ether or polyethylene glycol, preparable by mixing the constituents of the molded article in a mixer in such a manner that the frictional heat leads to the formation of fused granules which are pressed to give a molded article after cooling without further melting.

2. A molded article as claimed in claim 1, which contains at least one surface-active substance, a lubricant, a mold release agent and/or a substance which affects the mechanical properties of the preparation.

3. A molded article as claimed in claim 1 or 2, wherein the weighting agent is iron powder.

4. A molded article as claimed in one of claims 1 to 3, wherein the active substance is an agent for the control of parasitic infections and/or diseases, for the supply of deficient substances, for the control of metabolic processes or for the control of endocrine processes.

5. A molded article as claimed in one of claims 1 to 3, containing at least one active substance for improving growth and/or the utilization of feed.

6. A molded article as claimed in one of claims 1 to 4, wherein the active substance is an anthelmintic, preferably from the active substance group comprising benzimidazole and benzothiazole derivatives or the probenzimidazoles.

7. A molded article as claimed in claim 6, containing the active substance fenbendazole.

8. A molded article as claimed in one of claims 1 to 3 and 5, wherein the active substance is a polyether antibiotic.

9. A molded article as claimed in one of claims 1 to 3 and 5, wherein the active substance is a phosphoglycolipid.

10. A molded article as claimed in one of claims 1 to 3 and 5, containing the active substance salinomycin or its salt.

11. A molded article as claimed in claim 9, wherein the active substance is flavophospholipol.

12. A molded article for oral administration in ruminants as claimed in one of claims 1 to 11, which contains an adequate amount of the active substance for defined release during a fixed period, and optionally a coating which is soluble or can be degraded in the digestive tract.

13. A product containing a plurality of molded articles as claimed in claim 12.

14. A product as claimed in claim 13, wherein the molded articles are connected to one another by means of a tape, a thread, a chain, a rod, or by bonding or inclusion in a net.

15. A product as claimed in claim 13 or 14, which is composed of molded articles of varying dimension and shape, a different content of active substance and/or different type of active substance.

16. A product as claimed in one of claims 13 to 15, whose total volume is between 0.5 and 200 cm³.

17. A process for the preparation of a molded article as claimed in one of claims 1 to 11, which comprises preparing fused granules from the constituents of the molded article in a suitable mixer with utilization of the frictional heat, which fused granules are pressed to give a molded article after cooling without further melting.

18. A process for the preparation of molded articles as claimed in claim 12, fused granules prepared as claimed in claim 17 comminuting and pressing them to give the desired molded articles using a press and providing the molded articles thus obtained with a coating which is soluble or can be degraded in the digestive tract.

19. A molded article as claimed in claim 12 or a product as claimed in one of claims 13 to 16 for the prophylaxis and/or for the treatment of diseases in ruminants.

## Claims (Claims for the following Contracting State(s): ES, GR)

1. A process for the preparation of a molded article for oral administration in ruminants, containing 0.001 to 75% by weight of at least one active substance, 3 to 75% by weight of wax, 25 to 90% by weight of powdered weighting agent and 0 to 30% by weight of at least one physiologically tolerable sugar, sugar alcohol, water-soluble cellulose ether or polyethylene glycol, which comprises preparing fused granules from the constituents of the molded article in a suitable mixer with utilization of the frictional heat, which fused granules are pressed to give a molded article after cooling without further melting.

2. The process as claimed in claim 1, wherein a molded article is prepared which contains at least one surface-active substance, a lubricant, a mold release agent and/or a substance which affects the mechanical properties of the preparation.

3. The process as claimed in claim 1 or 2, wherein the weighting agent employed is iron powder.

4. The process as claimed in one of claims 1 to 3, wherein the active substance employed is an agent for the control of parasitic infections and/or diseases, for the supply of deficient substances, for the control of metabolic processes or for the control of endocrine processes.

5. The process as claimed in one of claims 1 to 3, in which the active substance employed is at least one active substance for improving growth and/or the utilization of feed.

6. The process as claimed in one of claims 1 to 4, wherein the active substance employed is an anthelmintic, preferably from the active substance group comprising benzimidazole and benzothiazole derivatives or the probenzimidazoles.

7. The process as claimed in claim 6, in which the active substance is fenbendazole.

8. The process as claimed in one of claims 1 to 3 and 5, wherein the active substance is a polyether antibiotic.

9. The process as claimed in one of claims 1 to 3 and 5, wherein the active substance is a phosphoglycolipid.

10. The process as claimed in one of claims 1 to 3 and 5, wherein the active substance is salinomycin or its salt.

11. The process as claimed in claim 9, wherein the active substance is flavophospholipol.

12. A process for the preparation of a molded article for oral administration in ruminants, as claimed in one of claims 1 to 11, which molded article contains an adequate amount of the active substance for defined release during a fixed period, which comprises providing the molded article with a coating which is soluble or can be degraded in the digestive tract.

13. The process as claimed in claim 12, in which a plurality of the molded articles thus prepared are connected to form a product.

14. The process as claimed in claim 13, wherein the molded articles are connected to one another by means of a tape, a thread, a chain, a rod, or by bonding or inclusion in a net.

15. The process as claimed in claim 13 or 14, wherein molded articles of varying dimension and shape, a different content of active substance and/or different type of active substance are employed.

16. The process as claimed in one of claims 13 to 15, wherein the total volume of the product thus produced is between 0.5 and 200 cm³.

## Revendications (Revendications pour l'(les) Etat(s) contractant(s) suivant(s): AT, BE, CH, DE, DK, FR, GB, IT, LI, LU, NL, PT, SE)

1. Composition moulée pour l'administration orale chez des ruminants, contenant de 0,001 à 75 % en poids d'au moins une substance active, de 3 à 75 % en poids de cire, de 25 à 90 % en poids d'un agent de lestage pulvérulent et de 0 à 30 % en poids d'au moins un sucre, sucre-alcool, éther de cellulose hydrosoluble ou polyéthyléneglycol physiologiquement acceptable, pouvant être préparée par mélange des composants de la composition moulée, dans un mélangeur, de manière que la chaleur de frottement conduise à la formation d'un produit granulé par fusion qui, après le refroidissement, est comprimé, sans fusion ultérieure, en une composition moulée.

2. Composition moulée selon la revendication 1, caractérisée en ce qu'elle contient au moins une substance tensioactive, un lubrifiant, un agent de démoulage et/ou une substance qui influe sur les propriétés mécaniques de la composition.

3. Composition moulée selon la revendication 1 ou 2, caractérisée en ce que l'agent de lestage est la poudre de fer.

4. Composition moulée selon l'une des revendications 1 à 3, caractérisée en ce que la substance active est un agent pour la lutte contre des maladies et/ou infections parasitaires, pour l'apport de substances rares, pour la régulation de processus métaboliques ou pour la régulation de processus endocriniens.

5. Composition moulée selon l'une des revendications 1 à 3, contenant au moins une substance active pour l'amélioration de la croissance et/ou de l'utilisation des aliments.

6. Composition moulée selon l'une des revendications 1 à 4, caractérisée en ce que la substance active est un anthelminthique, de préférence choisi dans le groupe des substances actives constitué par les benzimidazoles, les dérivés de benzothiazole ou les probenzimidazoles.

7. Composition moulée selon la revendication 6, contenant la substance active fenbendazole.

8. Composition moulée selon l'une des revendications 1 à 3, et 5, caractérisée en ce que la substance active est un antibiotique de type polyéther.

9. Composition moulée selon l'une des revendications 1 à 3, et 5, caractérisée en ce que la substance active est un phosphoglycolipide.

10. Composition moulée selon l'une des revendications 1 à 3, et 5, contenant la substance active salinomycine ou un de ses sels.

11. Composition moulée selon la revendication 9, caractérisée en ce que la substance active est le flavophospholipol.

12. Composition moulée pour l'administration orale chez des ruminants, selon l'une des revendications 1 à 11, qui contient une quantité suffisante de la substance active pour une libération définie pendant un temps déterminé, et éventuellement une enveloppe soluble ou dégradable dans le tube digestif.

13. Produit contenant plusieurs compositions moulées selon la revendication 12.

14. Produit selon la revendication 13, caractérisé en ce que les compositions moulées sont reliées entre elles au moyen d'une bande, d'un fil, d'une chaîne, d'une tige, par collage ou inclusion dans un réseau.

15. Produit selon la revendication 13 ou 14, caractérisé en ce qu'il consiste en compositions moulées de dimensions, formes différentes, ayant une teneur variable en substance active et/ou une nature variable de la substance active.

16. Produit selon l'une des revendications 13 à 15, dont le volume total est compris entre 0,5 et 200 cm³.

17. Procédé pour la préparation d'une composition moulée selon l'une des revendications 1 à 11, caractérisé en ce que l'on prépare, à partir des composants de la composition moulée, dans un mélangeur approprié, avec utilisation de la chaleur de frottement, un produit granulé par fusion qui, après le refroidissement, est comprimé, sans fusion ultérieure, en une composition moulée.

18. Procédé pour la préparation de compositions moulées selon la revendication 10, caractérisé en ce que l'on fragmente un produit granulé par fusion préparé selon la revendication 17, et on le comprime au moyen d'une presse en les compositions moulées recherchées, et on munit les compositions moulées ainsi obtenues d'une enveloppe soluble ou dégradable dans le tube digestif.

19. Composition moulée selon la revendication 12 ou produit selon l'une des revendication 13 à 16, pour la prophylaxie et/ou pour le traitement de maladies chez des ruminants.

## Revendications (Revendications pour l'(les) Etat(s) contractant(s) suivant(s): ES, GR)

1. Procédé pour la préparation d'une composition moulée pour l'administration orale chez des ruminants, contenant de 0,001 à 75 % en poids d'au moins une substance active, de 3 à 75 % en poids de cire, de 25 à 90 % en poids d'un agent de lestage pulvérulent et de 0 à 30 % en poids d'au moins un sucre, un sucre-alcool, éther de cellulose hydrosoluble ou polyéthyléneglycol physiologiquement acceptable, caractérisé en ce que l'on prépare, à partir des composants de la composition moulée, dans un mélangeur approprié, avec utilisation de la chaleur de frottement, un produit granulé par fusion qui, après le refroidissement, est comprimé, sans fusion ultérieure, en une composition moulée.

2. Procédé selon la revendication 1, caractérisé en ce que l'on prépare une composition moulée qui contient au moins une substance tensioactive, un lubrifiant, un agent de démoulage et/ou une substance qui influe sur les propriétés mécaniques de la composition.

3. Procédé selon la revendication 1 ou 2, caractérisé en ce que l'on utilise comme agent de lestage de la poudre de fer.

4. Procédé selon l'une des revendications 1 à 3, caractérisé en ce que l'on utilise comme substance active un agent pour la lutte contre des maladies et/ou infections parasitaires, pour l'apport de substances rares, pour la régulation de processus métaboliques ou pour la régulation de processus endocriniens.

5. Procédé selon l'une des revendications 1 à 3, dans lequel on utilise comme substance active au moins une substance active pour l'amélioration de la croissance et/ou de l'utilisation des aliments.

6. Procédé selon l'une des revendications 1 à 4, caractérisé en ce que l'on utilise comme substance active un anthelminthique, de préférence choisi dans le groupe des substances actives constitué par les benzimidazoles, les dérivés de benzothiazole ou les probenzimidazoles.

7. Procédé selon la revendication 6, dans lequel la substance active est le fenbendazole.

8. Procédé selon l'une des revendications 1 à 3, et 5, caractérisé en ce que la substance active est un antibiotique de type polyéther.

9. Procédé selon l'une des revendications 1 à 3, et 5, caractérisé en ce que la substance active est un phosphoglycolipide.

10. Procédé selon l'une des revendications 1 à 3, et 5, caractérisé en ce que la substance active est la salinomycine ou un de ses sels.

11. Procédé selon la revendication 9, caractérisé en ce que la substance active est le flavophospholipol.

12. Procédé pour la préparation d'une composition moulée pour l'administration orale chez des ruminants, selon l'une des revendications 1 à 11, qui contient une quantité suffisante de la substance active pour une libération définie pendant un temps déterminé, caractérisé en ce que l'on munit la composition moulée d'une enveloppe soluble ou dégradable dans le tube digestif.

13. Procédé selon la revendication 12, dans lequel on relie plusieurs des compositions moulées ainsi préparées en un produit.

14. Procédé selon la revendication 13, caractérisé en ce que l'on relie entre elles les compositions moulées au moyen d'une bande, d'un fil, d'une chaîne, d'une tige, par collage ou inclusion dans un réseau.

15. Procédé selon la revendication 13 ou 14, caractérisé en ce que l'on utilise des compositions moulées de dimensions, formes différentes, ayant une teneur variable en substance active et/ou une nature variable de la substance active.

16. Procédé selon l'une des revendications 13 à 15, caractérisé en ce que le volume total du produit ainsi préparé est compris entre 0,5 et 200 cm³.
